# DEMANDE DE BREVET EUROPEEN

(11) **EP 0 686 619 A1**
(43) Date de publication de la demande: **13.12.1995**
(21) Numéro de dépôt: 95401298.5
(22) Date de dépôt: 02.06.1995
(51) Int. Cl.: C07C 53/08, C07C 51/48

(54) **Procédé de récupération de l'acide acétique contenu dans un effluent en présence d'impuretés organiques**

(30) Priorité: 07.06.1994 FR 9406949
(71) Demandeur: COMMISSARIAT A L'ENERGIE ATOMIQUE, F-75015 Paris (FR)
(72) Inventeur: Leybros, Jean, F-30200 Bagnols/Ceze (FR); Morin, Michel, F-85800 L'Isle sur la Sorgue (FR)
(74) Mandataire: Dubois-Chabert, Guy

(57) **Abrégé**

L'invention concerne un procédé de récupération de l'acide acétique contenu dans un effluent en présence d'impuretés organiques.

Le but de l'invention est de récupérer un acide acétique très pur, de façon à pouvoir le recycler ou le valoriser ultérieurement.

Ce but est atteint à l'aide d'un procédé comprenant les étapes consistant à :
a) mettre, dans une colonne d'extraction (2), l'effluent (20) à traiter en contact avec un solvant organique (22, 22') d'extraction des impuretés organiques, ce solvant contenant essentiellement un hydrocarbure,
b) introduire dans une colonne d'entraînement à la vapeur (4), l'effluent (26) issu de la colonne d'extraction (2), afin d'éliminer les traces de solvant organique (22, 22'), et
c) récupérer en pied de la colonne d'entraînement à la vapeur (4), l'effluent traité (28) contenant l'acide acétique et débarrassé des impuretés organiques.

## Description

La présente invention concerne un procédé de récupération de l'acide acétique contenu dans un effluent en présence d'impuretés organiques.

Les procédés de synthèse organique que l'on rencontre par exemple dans l'industrie des terpènes, conduisent fréquemment à la formation d'effluents contenant de grandes quantités d'acide acétique, (jusqu'à 50% en masse) . Ces effluents contiennent également des impuretés organiques telles que des hydrocarbures, des alcools ou des esters. Dans l'industrie des terpènes, ces impuretés sont par exemple des hydrocarbures et des esters terpéniques.

Les effluents contenant de l'acide acétique présentent une demande chimique en oxygène (D.C.O.) élevée. La D.C.O. correspond à la quantité d'oxygène nécessaire à la dégradation chimique de ces effluents. Or, pour respecter les normes anti-pollution extrêmement strictes en vigueur, on ne peut rejeter dans l'environnement des effluents présentant une trop grande D.C.O. La quasi-totalité de la D.C.O. étant due principalement à l'acide acétique, il est nécessaire de séparer cet acide du reste des effluents qui poseront alors moins de problèmes de rejet dans l'environnement.

De nombreuses techniques de séparation ont été proposées et mises en oeuvre en fonction de la concentration en acide acétique de l'effluent. Seules quelques unes de ces techniques restent d'un usage industriel courant.

Dans le cas d'effluents contenant de fortes concentrations en acide acétique (supérieures à 50% en masse), la distillation fractionnée peut permettre d'obtenir un acide acétique très pur (qualité dite "acide glacial"). Toutefois, cette technique nécessite un grand nombre d'étages de séparation et un taux de reflux assez important pour obtenir le résultat escompté. En outre, il faut éviter que les impuretés présentes ne se répartissent sur toute la hauteur de la colonne de séparation.

Une autre technique peut être envisagée afin de réduire la consommation énergétique du procédé. Il s'agit de la déshydratation azéotropique. Cette technique met en oeuvre un additif dénommé "entraîneur", peu miscible dans l'eau et qui abaisse le point d'ébullition de l'eau par rapport à celui de l'acide acétique par la formation d'un azéotrope. Toutefois, ce procédé ne permet pas de séparer directement l'acide acétique de ces impuretés.

L'article de P. Eaglesfield et al., "Recovery of Acetic Acid from Dilute Aqueous Solutions by Liquid-Liquid Extraction", the Industrial Chemist, avril 1953, 147-151, décrit l'emploi de divers entraîneurs du type hydrocarbure, dichlorométhane, etc...

Dans le cas d'effluents présentant une concentration moyenne en acide acétique (comprise entre 5 et 50% en masse), les procédés actuellement connus sont l'extraction liquide-liquide avec un solvant organique, suivie d'une distillation azéotropique ou d'une déshydratation par distillation fractionnée.

Ces extractions liquide-liquide sont principalement réalisées à contre-courant dans des extracteurs qui peuvent être avantageusement du type colonne pulsée.

En outre, le raffinat contenant des traces d'acide acétique et une certaine quantité de solvant solubilisée, est traité par entraînement à la vapeur (connu sous la terminologie anglaise de "stripping") avant rejet.

Dans les procédés d'extraction liquide-liquide, les solvants utilisés sont classés en deux catégories par rapport au point d'ébullition de l'acide acétique qui est de 118°C. Ces deux catégories sont les solvants "lourds" dont le point d'ébullition est supérieur à 118°C et les solvants "légers" dont le point d'ébullition est inférieur à 118°C, suivant que ce solvant est récupéré respectivement en pied ou en tête de la colonne de distillation.

Lorsque l'on utilise un solvant "léger", l'effluent à traiter est envoyé dans une colonne d'extraction à contre-courant avec un solvant. Le solvant est récupéré en tête d'une colonne de distillation sous forme d'un hétéroazéotrope composé du solvant et d'eau. Après une étape de condensation et de décantation, le solvant est recyclé en direction de la colonne d'extraction. Le raffinat d'extraction est dirigé vers une colonne d'entraînement à la vapeur des raffinats, afin d'éliminer les traces de solvant solubilisé.

Dans le cas des effluents à faible concentration en acide acétique (inférieurs à 10% en masse), on utilise un solvant "lourd". Dans ce cas, l'effluent à traiter est aussi envoyé dans une colonne d'extraction. L'extrait est soumis à une étape de déshydratation par distillation. L'extrait déshydraté récupéré au pied de la colonne de déshydratation est alors envoyé dans une colonne de régénération afin de subir un traitement de fractionnement. En tête de la colonne de fractionnement (colonne de régénération), on récupère l'acide acétique brut. Ce procédé peut permettre de réduire les besoins en vapeur nécessaires à la régénération du solvant puisque l'acide acétique et l'eau extraite dont la quantité à vaporiser est plus faible, passent en tête de la colonne de distillation.

Généralement, la valorisation de l'acide acétique n'est possible que si celui-ci est récupéré sous une forme très pure (qualité acide glacial) . Or, dans la plupart des techniques précitées, les impuretés sont souvent co-extraites en même temps que l'acide acétique. Ainsi, dans le procédé utilisant un solvant "léger" il est très difficile de purifier l'acide acétique obtenu en pied de la colonne de distillation, car il contient des impuretés à haut point d'ébullition. De manière analogue avec le procédé utilisant un solvant "lourd", l'acide acétique obtenu en tête de la colonne de distillation contient des impuretés à bas point d'ébullition et, ... il existe un risque d'accumulation, dans le solvant, d'impuretés à haut point d'ébullition co-extraites par ce procédé. Ceci nécessite des purges continues ou périodiques d'une partie du solvant. En conséquence, les coûts d'exploitation sont considérablement augmentés.

Dans l'industrie des terpènes, par exemple, les impuretés sont constituées par un mélange d'hydrocarbures et d'esters terpéniques. Le traitement de tels effluents par l'un des procédés précités pose des problèmes car lors des distillations nécessaires à l'obtention de l'acide acétique pur du type acide glacial, on observe une partition des impuretés sur toute la hauteur de la colonne de distillation, ce qui ne permet pas d'obtenir un acide acétique de bonne qualité. En outre, on peut observer un phénomène d'accumulation de ces impuretés dans le solvant.

L'invention a pour but de remédier à ces problèmes et de réaliser un procédé permettant de récupérer l'acide acétique, en vue de sa valorisation ou de son recyclage ultérieur, à partir d'un effluent contenant simultanément de l'acide acétique et des impuretés organiques.

A cet effet, l'invention concerne un procédé de récupération d'acide acétique contenu dans un effluent en présence d'impuretés organiques. Selon les caractéristiques de l'invention, ce procédé comprend les étapes consistant à :
a) mettre, dans une colonne d'extraction, l'effluent à traiter en contact avec un solvant organique d'extraction des impuretés organiques, ce solvant contenant essentiellement un hydrocarbure,
b) introduire dans une colonne d'entraînement à la vapeur, l'effluent issu de la colonne d'extraction afin d'éliminer les traces de solvant organique, et
c) récupérer en pied de la colonne d'entraînement à la vapeur, l'effluent traité contenant l'acide acétique et débarrassé des impuretés organiques.

De façon avantageuse, le solvant organique est un hydrocarbure aliphatique ou aromatique dont le nombre de carbone est compris entre 6 et 10.

L'emploi d'un hydrocarbure dans le procédé selon l'invention présente de nombreux avantages. Premièrement, les coeffients de partage (Kd) sont tels que la quasi-totalité des impuretés est extraite de l'effluent alors que l'acide acétique l'est peu. Deuxièmement, les hydrocarbures sont très peu solubles dans l'eau et forment généralement avec celle-ci des azéotropes qui facilitent l'élimination des traces de solvant, dissoutes dans l'effluent acide lors de son traitement dans la colonne d'entraînement à la vapeur.

De façon avantageuse, l'hydrocarbure utilisé est de l'hexane (Kd>10), du fait de sa sélectivité pour les impuretés vis-à-vis de l'acide acétique (Kd<0,02), de sa très faible solubilité dans l'eau (<10 mg/l) et de ses azéotropes à bas point d'ébullition (hexane/acide acétique, Eb=68,3°C ; hexane/eau Eb=61,5°C).

De façon avantageuse, un condensat contenant de l'acide acétique, des impuretés organiques et du solvant organique et issu de la tête de la colonne d'entraînement à la vapeur est recyclé dans la colonne d'extraction avec l'effluent à traiter entrant. Ceci permet d'éviter les pertes en solvant, acide acétique et impuretés tout particulièrement si ces dernières sont valorisables.

Enfin, selon une variante de réalisation de l'invention, le solvant organique chargé en impuretés organiques et issu de la colonne d'extraction est introduit dans une colonne de distillation, de façon à récupérer en tête de ladite colonne de distillation, le solvant propre et en pied de colonne, une solution concentrée d'impuretés. Du fait que les hydrocarbures du solvant organique forment généralement un azéotrope avec l'acide acétique, ceci facilite la concentration des impuretés à la sortie de la colonne de distillation sans perte d'acide acétique.

Dans l'industrie des terpènes par exemple, le passage de l'effluent traité contenant l'acide acétique dans une colonne d'entraînement à la vapeur conformément à l'invention présente l'avantage d'éliminer les dernières traces d'impuretés qui peuvent elles-mêmes être entraînées ultérieurement (formation d'azéotropes avec la vapeur d'eau). De plus, la génération de la vapeur d'eau à partir de l'effluent lui-même permet d'éviter la dilution de l'effluent.

Dans l'industrie des terpènes, les impuretés organiques sont généralement constituées par un mélange d'hydrocarbures et d'esters terpéniques. Ces impuretés, une fois recueillies à la sortie de la colonne de distillation, sont valorisables soit sous la forme d'un concentré dans l'hydrocarbure d'extraction, soit sous la forme de substitut des chlorofluorocarbones et des solvants chlorés. De tels substituts peuvent être utilisés dans l'industrie du traitement de surface pour le dégraissage des pièces métalliques.

La valorisation de la totalité des produits présents dans l'effluent est une caractéristique particulièrement avantageuse qui peut être obtenue grâce au procédé selon l'invention. En effet, dans les techniques de l'art antérieur, l'acide acétique impur obtenu était généralement détruit par incinération.

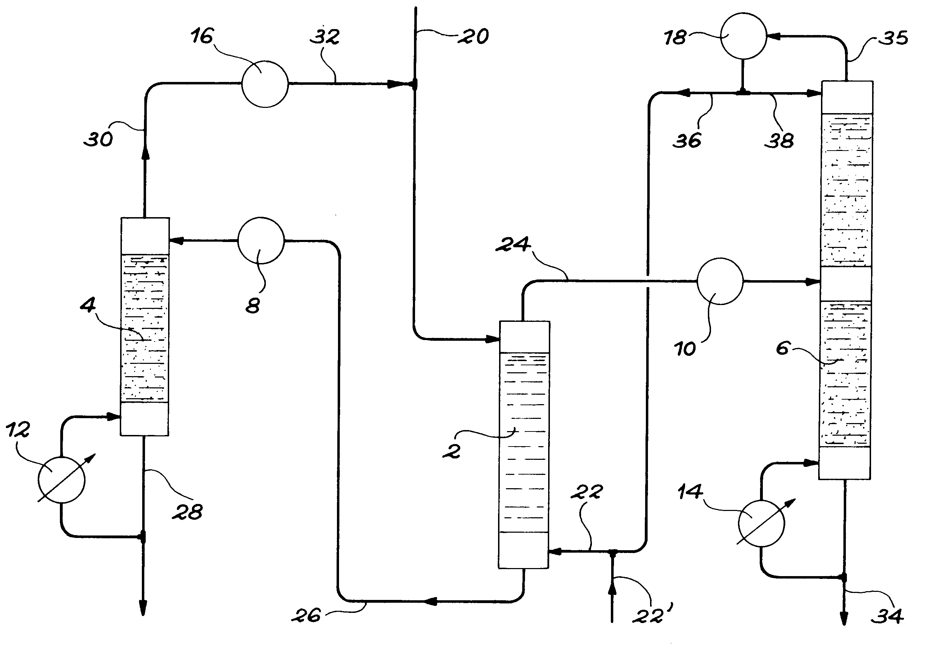

D'autres caractéristiques et avantages de l'invention ressortiront mieux à la lecture de la description suivante, donnée à titre d'exemple illustratif et non limitatif, cette description étant faite en faisant référence au dessin joint.

Comme illustré sur la figure, le dispositif utilisé pour la mise en oeuvre du procédé selon l'invention comprend une colonne d'extraction 2 de préférence du type liquide-liquide, une colonne d'entraînement à la vapeur 4 et une colonne de distillation 6. Un échangeur de chaleur 8 est placé sur une canalisation reliant le pied de la colonne d'extraction liquide-liquide 2 à la tête de la colonne d'entraînement à la vapeur 4. En outre, un autre échangeur de chaleur 10 est placé sur la canalisation reliant la tête de la colonne d'extraction 2 et l'entrée de la colonne de distillation 6.

Deux rebouilleurs 12 et 14 sont prévus respectivement au pied de la colonne d'entraînement à la vapeur 4 et au pied de la colonne de distillation 6.

Enfin, un échangeur de chaleur 16 est prévu à la sortie de la colonne d'entraînement à la vapeur 4 et un autre échangeur de chaleur 18 est prévu à la tête de la colonne de distillation 6.

Le fonctionnement du dispositif va maintenant être décrit de façon détaillée.

L'effluent à traiter 20 contenant de l'acide acétique et des impuretés organiques est introduit en tête de la colonne d'extraction 2, à contre-courant d'un solvant organique d'extraction 22 introduit au pied de ladite colonne. Cette opération est réalisée à température ambiante. Cette colonne permet de réaliser l'élimination des impuretés organiques. Le solvant organique 24 chargé en impuretés organiques sort en tête de la colonne d'extraction 2, tandis que l'effluent 26 contenant l'acide acétique est évacué en pied de cette colonne 2.

Dans cette colonne d'extraction 2, le rapport du débit volumique de la phase aqueuse par rapport à celui de la phase organique est de préférence supérieur ou égal à 3, afin d'effectuer une première concentration des impuretés. Ce rapport est toujours adapté à la quantité d'impuretés à extraire. L'effluent 26 issu de la colonne d'extraction 2 est introduit après préchauffage au moyen de l'échangeur de chaleur 8 dans la colonne d'entraînement à la vapeur 4. A l'intérieur de celle-ci, il circule à contre-courant de la vapeur créée à partir de l'effluent lui-même, au moyen d'un rebouilleur 12. Ceci permet d'éviter la dilution de l'effluent à traiter.

L'effluent traité 28 sortant au pied de la colonne d'entraînement à la vapeur 4 contient la quasi-totalité de l'acide acétique présent au départ dans l'effluent à traiter et est par contre débarrassé de toutes les impuretés organiques et des traces de solvant. Généralement, cet effluent est dirigé vers une ligne de récupération classique de l'acide acétique.

Par ailleurs, les vapeurs 30 constituées d'eau, de solvant organique et d'acide acétique sortant en tête de la colonne d'entraînement à la vapeur 4 sont condensées par un échangeur de chaleur 16 puis les condensats 32 obtenus sont retournés en tête de la colonne d'extraction 2, après mélange avec l'effluent à traiter 20.

De préférence, dans la colonne d'entraînement à la vapeur 4, le rapport du débit massique de la phase liquide par rapport à celui de la phase vapeur est supérieur ou égal à 15. Ceci permet de ne pas trop diluer l'effluent à traiter 20 entrant dans la colonne d'extraction 2, lors du recyclage du condensat 32 avec l'effluent à traiter 20.

Le solvant organique 24 chargé en impuretés organique et issu de la tête de la colonne d'extraction 2 est traité par distillation dans la colonne de distillation 6 après un préchauffage au moyen de l'échangeur de chaleur 10. Une solution concentrée 34 constituée par des impuretés organiques et par une fraction du solvant organique est récupérée à la base du rebouilleur 14 de la colonne de distillation 6.

En outre, les vapeurs organiques 35 constituées de solvant organique et d'acide acétique résiduel sortant en tête de la colonne de distillation 6 sont condensés par l'échangeur de chaleur 18. A la sortie de cet échangeur de chaleur 18, les condensats 36 sont retournés au pied de la colonne d'extraction 2 au point où est introduit le solvant organique 22. Le reflux 38 est prélevé et retourné vers la colonne de distillation 6.

De façon avantageuse, un appoint de solvant 22' est réalisé pour compenser la fraction du solvant sortant dans la solution concentrée d'impuretés 34 issue de la colonne de distillation 6.

Selon le premier mode de réalisation du procédé selon l'invention, le dispositif ne comprend que la colonne d'extraction 2 et la colonne d'entraînement à la vapeur 4 et les échangeurs de chaleur et rebouilleurs qui y sont associés.

Selon un second mode de réalisation dans lequel le solvant utilisé est recyclé, l'installation comprend la totalité des installations représentées sur la figure jointe.

On donnera ci-après un exemple de mise en oeuvre du procédé conforme à l'invention.

### Exemple:

Dans une installation analogue à celle qui vient d'être décrite, on traite un effluent 20 provenant d'un procédé de synthèse organique de la chimie des terpènes. L'effluent 20 et le solvant organique 22 sont introduits dans la colonne d'extraction 2 avec un débit de solvant correspondant à 20% du débit d'effluent. L'opération d'extraction est réalisée à la température de 25°C. Le tableau 1 ci-après donne la composition des différents effluents et solutions au fur et à mesure de leur traitement par le procédé selon l'invention.

**Tableau 1**

| Nature de l'effluent | effluent à traiter (20) | condensat (32) issu de la colonne d'entraînement (4) | solvant chargé en impuretés (24) | solution concentrée en impuretés (34) | condensats (36) |
|---|---|---|---|---|---|
| acide acétique (g/l) | 304 | 206 | 5,9 | - | 309 |
| impuretés (g/l) | 4,66 | 1,2 | 18,85 | 188,85 | 0,06 |
| solvant organique (mg/l) | - | 71 | - | - | 5,2 |
| eau (g/l) | - | - | 0,11 | - | - |

Après le traitement de l'effluent 26 dans la colonne d'entraînement à la vapeur 4, l'effluent 28 traité par un débit massique de vapeur correspondant à 5% du débit liquide est récupéré. Il contient 309 g d'acide acétique par litre de solution tandis que la quantité d'impuretés résiduelles est inférieure aux limites de détection (<10 mg/l).

L'effluent purifié 28 peut ensuite avantageusement être traité par un procédé classique à l'acétate d'éthyle, par exemple, et conduire à l'obtention d'un acide acétique de qualité "acide glacial".

## Revendications

1. Procédé de récupération d'acide acétique contenu dans un effluent en présence d'impuretés organiques, caractérisé en ce qu'il comprend les étapes consistant à :
a) mettre, dans une colonne d'extraction (2), l'effluent (20) à traiter en contact avec un solvant organique (22, 22') d'extraction des impuretés organiques, ce solvant contenant essentiellement un hydrocarbure,
b) introduire dans une colonne d'entraînement à la vapeur (4), l'effluent (26) issu de la colonne d'extraction (2), afin d'éliminer les traces de solvant organique (22, 22'), et
c) récupérer en pied de la colonne d'entraînement à la vapeur (4), l'effluent traité (28) contenant l'acide acétique et débarrassé des impuretés organiques.

2. Procédé de récupération selon la revendication 1, caractérisé en ce que le solvant organique (22, 22') est un hydrocarbure aliphatique ou aromatique dont le nombre de carbone est compris entre 6 et 10.

3. Procédé de récupération selon la revendication 2, caractérisé en ce que le solvant organique (22, 22') est de l'hexane.

4. Procédé de récupération selon la revendication 1, caractérisé en ce que la vapeur d'eau utilisée dans la colonne d'entraînement à la vapeur (4) est obtenue à partir de l'effluent lui-même, au moyen d'un rebouilleur (12) placé au pied de ladite colonne d'entraînement.

5. Procédé de récupération selon la revendication 1, caractérisé en ce qu'un condensat (32) contenant de l'acide acétique, des impuretés organiques et du solvant organique et issu de la tête de la colonne d'entraînement à la vapeur (4) est recyclé dans la colonne d'extraction (2).

6. Procédé de récupération selon la revendication 1, caractérisé en ce que dans la colonne d'extraction (2), le rapport du débit volumique de la phase aqueuse par rapport à celui de la phase organique est supérieur ou égal à 3.

7. Procédé de récupération selon la revendication 1, caractérisé en ce que dans la colonne d'entraînement à la vapeur (4), le rapport du débit massique de la phase liquide par rapport à celui de la phase vapeur est supérieur ou égal à 15.

8. Procédé de récupération selon la revendication 1, caractérisé en ce que le solvant organique chargé en impuretés organiques (24) et issu de la colonne d'extraction (2) est introduit dans une colonne de distillation (6), de façon à récupérer en tête de ladite colonne de distillation, le solvant propre (35, 36) et en pied de colonne, une solution concentrée d'impuretés (34).

9. Procédé de récupération selon la revendication 8, caractérisé en ce que le solvant propre (35, 36) issu de la colonne de distillation (6) est recyclé dans la colonne d'extraction (2).
